Europäisches Patentamt

European Patent Office

Office européen des brevets

(19)

(11) **EP 1 003 538 B1**

(12) **FASCICULE DE BREVET EUROPEEN**

(45) Date de publication et mention
de la délivrance du brevet:
**18.02.2004 Bulletin 2004/08**

(21) Numéro de dépôt: **98942719.0**

(22) Date de dépôt: **10.08.1998**

(51) Int Cl.7: **A61K 35/78**, A61K 7/48

(86) Numéro de dépôt international:
**PCT/FR1998/001784**

(87) Numéro de publication internationale:
**WO 1999/007397 (18.02.1999 Gazette 1999/07)**

(54) **UTILISATION D'EXTRAITS DE POLYGONATUM POUR STIMULER LA SYNTHESE DE L'ELASTINE CUTANEE**

VERWENDUNG VON POLYGONATUM EXTRAKTEN, UM DIE HAUTELASTIN-SYNTHESE ZU STIMULIEREN

USE OF POLYGONATUM EXTRACTS FOR STIMULATING DERMAL ELASTIN SYNTHESIS

(84) Etats contractants désignés:
**DE ES FR GB IT**

(30) Priorité: **11.08.1997 FR 9710258**

(43) Date de publication de la demande:
**31.05.2000 Bulletin 2000/22**

(73) Titulaire: **LVMH RECHERCHE**
**75008 Paris (FR)**

(72) Inventeurs:
• **MEYBECK, Alain**
**F-92400 Courbevoie (FR)**
• **BONTE, Frédéric**
**F-45100 Orléans (FR)**
• **DUMAS, Marc**
**F-45100 Orléans (FR)**

(74) Mandataire: **Giraud, Françoise et al**
**Cabinet Beau de Loménie**
**158, rue de l'Université**
**75340 Paris Cedex 07 (FR)**

(56) Documents cités:
• **PATENT ABSTRACTS OF JAPAN vol. 097, no. 002, 28 février 1997 & JP 08 283172 A (KOSE CORP), 29 octobre 1996**
• **PATENT ABSTRACTS OF JAPAN vol. 018, no. 236 (C-1196), 6 mai 1994 & JP 06 024937 A (NARISU KESHOHIN:KK), 1 février 1994**
• **PATENT ABSTRACTS OF JAPAN vol. 017, no. 216 (C-1053), 28 avril 1993 & JP 04 356416 A (KOSE CORP), 10 décembre 1992**
• **DATABASE WPI Section Ch, Week 9139 Derwent Publications Ltd., London, GB; Class B04, AN 91-286062 XP002062784 & JP 03 190809 A (SHISEIDO CO LTD), 20 août 1991**

**Description**

La présente invention concerne l'utilisation d'extraits de Polygonatum pour stimuler la synthèse de l'élastine cutanée.

Dans ce but, elle concerne notamment l'utilisation d'extraits de Polygonatum kingianum Coll. Et Hemsl, Polygonatum sibiricum Redoute, Polygonatum cyrtonema Hua, Polygonatum zanlanscianense, et plus particulièrement, l'utilisation d'extraits des rhizomes de Polygonatum connus sous le nom de Huang Jing (Liu Y.P. et al, Shoyakugaku Zasshi 47 (3), 1993, 249-266) Dans la suite de la présente description, ces différents extraits sont désignés sous le nom générique d'extraits de Polygonatum par simplification.

Les rhizomes de Polygonatum et, notamment de Polygonatum sibiricum Redoute, de Polygonatum cynonema Hua ou de Polygonatum kingianum Coll. et Hemsl. (Liliacées) sont utilisés en médecine traditionnelle chinoise sous le nom de Huang Jing (Matière Médicale Chinoise - B. Auteroche, M. Auteroche, M. Demont - Maloine 1992, p. 620). Les principales indications des rhizomes de Polygonatum sont pour le traitement des toux sèches ducs à la déficience du poumon, pour tonifier les reins ou pour traiter les déficiences de la rate ou de l'estomac.

Par ailleurs, il est connu par l'article de Grégory C. Sephel et al. dans J. invest. Dermatol. 1986, 86, pages 279-285, que la production d'élastine par les fibroblastes de donneurs âgés diminue. Il est également connu que l'élasticité cutanée diminue de façon significative avec l'âge comme décrit par Takema et al, dans British Journal of Dermatology 1994, 131, pages 641-648.

Dans le cadre de l'invention, il a été mis en évidence, de manière inattendue, que des extraits de Polygonatum permettaient de stimuler la synthèse d'élastine par les fibroblastes du derme, en particulier les fibroblastes du derme humain.

Or, on rappellera que dans le derme, les fibres élastiques forment un réseau qui contribue à l'élasticité de la peau et par conséquent à sa tonicité et à sa fermeté. L'élastine est le composant majeur de ces fibres. Elle est initialement synthétisée par les fibroblastes sous la forme d'un polypeptide soluble de 70 kDa. la tropoélastine. La formation des microfibrilles se réalise ensuite par des liaisons intramoléculaires appelées desmosines entre les résidus lysine des chaines polypeptidiques. Ces liaisons confèrent à l'élastine une grande insolubilité.

Ainsi, la présente invention a pour but de résoudre le nouveau problème technique consistant en la fourniture d'une solution permettant d'améliorer l'élasticité cutanée en favorisant la synthèse d'élastine, selon une solution de conception simple, aisément utilisable à l'échelle industrielle tout en permettant un usage cosmétique ou pharmaceutique, notamment dermatologique.

Ainsi, la présente invention concerne l'utilisation d'un extrait de Polygonatum comme agent cosmétique destiné à stimuler la synthèse d'élastine par les fibroblastes du derme.

Dans cette utilisation, la stimulation de la synthèse de l'élastine a pour résultat d'améliorer l'élasticité et la tonicité cutanées ou de favoriser le raffermissement cutané.

Dans cette utilisation, on choisira avantageusement l'extrait parmi les extraits de rhizomes de Polygonatum kingianum, de P. sibiricum, de P. cyrtonema, de P. zanlanscianense et les autres extraits de rhizomes de Polygonatum connus sous le nom chinois de Huan Jing.

L'extrait de Polygonatum précité peut être utilisé aussi bien à titre curatif que préventif, pour corriger ou prévenir la perte d'élasticité, de tonicité ou de fermeté cutanée, due notamment au vieillissement ou à l'exposition aux rayons ultraviolets.

Selon un mode de réalisation avantageux de l'invention, l'extrait de Polygonatum est un extrait des rhizomes broyés.

L'extrait est avantageusement obtenu par un procédé mettant en oeuvre une étape d'extraction de la matière végétale par un solvant polaire ou un mélange de solvants polaires.

A titre d'exemple nullement limitatif, on réalise l'extraction au moyen d'un solvant choisi dans le groupe constitué par l'eau, les alcools comprenant de préférence de 1 à 4 atomes de carbone, et les solvants mixtes à base de mélanges quelconques des solvants précités.

De préférence, on utilisera un alcool tel que le méthanol, l'éthanol, le propanol, l'isopropanol, le propylèneglycol ou le butylèneglycol, ou un mélange de ces alcools ou encore un mélange hydro-alcoolique.

On choisira avantageusement comme solvant pour réaliser cette extraction un solvant choisi dans le groupe constitué par le méthanol, l'éthanol, le 1,3-butylèneglycol et les mélanges de ces solvants entre eux ou avec l'eau.

Dans ce cadre, les procédures d'extraction classiques bien connues de l'homme de l'art peuvent être utilisées. En particulier, l'extraction peut être réalisée sur la matière végétale broyée. Avantageusement, l'extraction est réalisée à partir des rhizomes. La matière végétale broyée est introduite dans le solvant d'extraction, de préférence constitué par le solvant ou le mélange de solvants polaires précités. L'extraction peut être renouvelée plusieurs fois jusqu'à épuisement de la matière végétale, conformément aux procédés bien connus de l'homme de l'art. L'extraction peut être réalisée à la température ambiante, ou à chaud et notamment au reflux du solvant. La proportion en poids entre la matière végétale et le solvant peut varier dans de larges limites. Plus précisément, elle peut être comprise entre 1 :

5 et 1 : 100, et, de préférence, entre 1 : 10 et 1 : 20.

Selon un autre mode de réalisation avantageux, l'extrait de Polygonatum précité est utilisé par voie topique à une concentration comprise entre 0,001 % et 10 % par rapport au poids total d'une composition le contenant dans un excipient, véhicule ou support approprié, de préférence cosmétiquement acceptable. Une concentration préférée est comprise entre 0,01 et 2 % en poids par rapport au poids total de la composition le contenant.

Selon un mode de réalisation avantageux de l'invention, l'extrait de Polygonatum selon l'invention est utilisé en combinaison avec une quantité efficace d'un principe actif stimulant la synthèse des composants de la matrice extracellulaire du derme, parmi lesquels le collagène, en particulier le collagène 1 et le collagène III, et les glycosaminoglycanes, en particulier l'acide hyaluronique.

On utilise, à titre d'exemple de principe actif stimulant la synthèse du collagène, en particulier du collagène I et du collagène III, le madécassoside, un extrait de Centella asiatica, ou un extrait de ginseng, en particulier un extrait contenant le ginsénoside $R_o$, ledit principe actif étant à une concentration comprise entre 0,01 % et 5 % en poids par rapport au poids total de la composition finale.

On utilise, à titre d'exemple de principe actif stimulant la synthèse des glycosaminoglycanes, un extrait végétal tel qu'un extrait de Filicium decipiens, en particulier un extrait d'écorce de racine de cette plante, décrit dans la demande de brevet français 95 07708, ou un extrait d'Eryobotria japonica décrit dans la demande de brevet français 96. 00018, ou un facteur de croissance, en particulier le PDGF (platelet derived growth factor), facteur de croissance dérivé des plaquettes sanguines.

Selon un autre mode de réalisation avantageux, l'extrait de Polygonatum précité est utilisé en combinaison avec une vitamine, en particulier la vitamine A, son ester palmitique, propionique ou acétique ou la vitamine E et ses dérivés. en particulier son phosphate, à une concentration comprise entre 0,0001 et 10 %, de préférence entre 0,0001 et 5 %, en poids par rapport au poids total de la composition.

Selon un troisième aspect, la présente invention couvre aussi une méthode de traitement, cosmétique de la peau, destinée à prévenir ou à corriger une perte d'élasticité, de tonicité ou de fermeté cutanée, caractérisée en ce qu'elle comprend l'application sur les zones de la peau à traiter d'une quantité efficace d'un extrait de Polygonatum, à titre de principe actif, pour stimuler la synthèse d'élastine par les fibroblastes, ledit principe actif étant incorporé dans un excipient cosmétiquement acceptable.

Pour la mise en oeuvre d'un tel procédé, on choisira avantageusement un extrait de rhizome de Polygonatum kingianum, de P. sibiricum, de P. cyrtonema, de P. zanlanscianense, ou tout autre extrait de rhizome de Polygonatum connu sous le nom chinois de Huang Jing.

Les variantes de réalisation de cette méthode résultent également clairement des variantes de réalisation de l'utilisation précitée.

Il résulte de ce qui précède que les extraits de Polygonatum selon l'invention sont précieux pour la fabrication d'une composition cosmétique où l'on recherche une amélioration de l'élasticité cutanée.

L'homme de l'art comprend ainsi l'intérêt majeur de l'invention qui favorise la synthèse d'élastine et sa facilité d'emploi grâce à une application topique sur la peau.

D'autres buts, caractéristiques et avantages de l'invention apparaîtront clairement à la lumière de la description explicative qui va suivre faite en référence aux exemples donnés ci-après simplement à titre d'illustration et qui ne sauraient donc en aucune façon limiter la portée de l'invention.

Dans les exemples, tous les pourcentages sont donnés en poids sauf indication contraire.

Exemple 1

Préparation d'un extrait méthanolique de rhizomes de Polygonatum kingianum :

On traite à reflux pendant 30 minutes, 100 g de rhizomes séchés et broyés de Polygonatum kingianum avec 1 litre de méthanol.

On laisse refroidir la phase méthanolique jusqu'à température ambiante, puis on filtre. Le résidu solide est extrait une nouvelle fois de la même façon avec 1 litre de méthanol.

On réunit les deux filtrats méthanoliques et on évapore le solvant sous vide au ballon rotatif jusqu'à l'obtention d'un résidu solide.

On obtient ainsi l'extrait méthanolique de Polygonatum kingianum, ci-après appelé E1.

Exemple 2

Préparation d'un extrait hydro-éthanolique de rhizomes de Polygonatum sibiricum:

On procède comme à l'exemple 1, si ce n'est que la matière végétale est du rhizome de Polygonatum sibiricum et que le solvant est un mélange éthanol-eau 75 : 25 en volume.

De plus, l'extrait obtenu après évaporation du solvant au ballon rotatif est lyophilisé pour obtenir un produit sec pulvérulent.

Exemple 3

Exemple d'essai démontrant l'activité d'un extrait de Polygonatum sur la stimulation de la synthèse de l'élastine par des fibroblastes cutanés humains

Matériel et méthodes

Provenance des cellules :

On utilise dans cette étude une souche de fibroblastes humains normaux (FHN) provenant de peau chirurgicale du visage d'une femme de 47 ans ainsi qu'une souche de FHN provenant de peau chirurgicale de la poitrine d'une femme de 49 ans. Les cellules sont obtenues par la méthode des explants telle que décrite par Dumas M. et al. dans l'article ayant pour titre "in vitro biosynthesis of type 1 and III collagens by human dermal fibroblasts from donors of increasing ages" dans Mech. Ageing Dev. <u>73</u> (1994), pages 179-187.

Mise en oeuvre des cultures de cellules :

Ces fibroblastes cutanés humains normaux ainsi obtenus sont ensemencés dans les puits d'une boite de culture multipuits, dans un milieu de culture E 199, dans lequel on a ajouté 2 millimoles par litre de L-glutamine et 25 micromoles par litre d'ascorbate de sodium.

24 heures après l'ensemencement, certains puits reçoivent de l'extrait de Polygonatum à 1 ou 2,5 ou 10 $\mu$g/ml par l'intermédiaire d'une solution dans le DMSO de façon à ce que la concentration finale de DMSO soit de 0.1 %.

On utilise à chaque fois six puits par produit et par concentration, ainsi que six puits ne recevant aucun produit à tester pour les cultures témoins.

Ces cultures témoins ne reçoivent que du DMSO à la concentration finale de 0,1 % en poids.

La culture est poursuivie pendant 48 h.

Après 48 h de culture comme indiqué ci-dessus, on dose sur les surnageants de la culture l'élastine sécrétée par une méthode ELISA dérivée de celle utilisée pour le dosage du collagène comme décrit par Dumas M. et al. dans la publication citée ci-dessus, à ceci près qu'un anticorps polyclonal anti-élastine humaine du commerce a été utilisé à la place de l'anticorps polyclonal anti-collagène 1 et III humain.

Ce dosage est naturellement réalisé aussi sur des cultures témoins, ne recevant pas de produit à tester.

Une gamme étalon réalisée à partir d'élastine soluble du commerce permet de convertir en nanogramme d'élastine les valeurs de densité optique (DO) obtenues à partir des surnageants de culture.

Dosage des protéines :

Parallèlement au dosage de l'élastine, un dosage de protéines cellulaires est effectué sur les cultures de fibroblastes, après élimination du surnageant, afin de ramener la quantité d'élastine dosée à une quantité fixe de protéines cellulaires. Le dosage est effectué en utilisant le kit BCA-1 (Bicinchoninic acid kit, commercialisé par Sigma, France) après dissolution du tapis cellulaire par de la soude 0,1 N.

Expression des résultats et statistiques :

Les résultats sont exprimés en nanogrammes d'élastine sécrétée par microgramme de protéines cellulaires. L'activité A de stimulation, exprimée en pourcentage, est calculée selon la formule suivante :

$$A = \frac{q - q_o}{q_o} \times 100$$

dans laquelle :

> q représente la quantité d'élastine sécrétée par les FHN traités,
> $q_o$ représente la quantité d'élastine secrétée par les FHN témoins.

Les résultats obtenus sur les cultures traitées (n=6) et témoins (n=6) sont comparés à l'aide du test t de Student pour séries non appariées. Toute valeur de p < 0,05 indiquera qu'une différence significative existe entre la sécrétion d'élastine des FHN témoins et ceux traités avec les produits de l'invention.

Les tableaux 1 et 2 rapportent les résultats d'essai avec l'extrait de Polygonatum obtenu selon l'exemple 1 à différentes concentrations sur les cultures de fibroblastes des deux donneurs.

TABLEAU 1

| Activité d'un extrait de Polygonatum sur la synthèse de l'élastine (fibroblastes du visage d'une femme de 47 ans) | | | |
|---|---|---|---|
| Conditions expérimentales | Elastine sécrétée ng/µg prot/ 48 h ± σ * | Activité A en % | Test t de Student valeurs de p |
| Témoin (Asc 25 µM + DMSO 0.1 %) | 11 ± 1,6 | 0 | |
| Extrait de l'exemple 1 à 1 µg/ml | 12,5 ± 1,3 | 14 | 0,063 |
| Extrait de l'exemple 1 à 2,5 µg/ml | 12,9 ± 2,9 | 17 | 0,19 |
| Extrait de l'exemple 1 à 10 µg/ml | 13,9 ± 2,16 | 26 | 0,02 |

TABLEAU 2

| Activité d'un extrait de Polygonatum sur la synthèse de l'élastine (fibroblastes du visage d'une femme de 49 ans) | | | |
|---|---|---|---|
| Conditions expérimentales | Elastine sécrétée ng/µg prot/ 48 h ± σ * | Activité A en % | Test t de Student valeurs de p |
| Témoin (Asc 25 µM + DMSO 0,1 %) | 18,1 ± 2,8 | 0 | |
| Extrait de l'exemple 1 à 10 µg/ml | 24,9 ± 3,3 | 38 | 0.004 |

σ = écart type ou déviation standard

Conclusion

Comme il ressort des tableaux 1 et 2, l'extrait de Polygonatum stimule fortement la synthèse d'élastine. Cette stimulation augmente avec la concentration du produit testé.

Il est donc démontré que l'utilisation d'un extrait de Polygonatum permet de stimuler la synthèse d'élastine par les fibroblastes de manière significative et particulièrement inattendue.

Exemples d'applications

Les compositions sont exprimées ci-dessous en pourcentages en poids.

Exemple 4

| Gel aux liposomes pour le soin du visage : | | |
|---|---|---|
| extrait de Polygonatum selon l'exemple 1 | | 0,2 |
| lécithine de soja | | 1 |
| acide hyaluronique en solution aqueuse à 1% | | 1 |
| Carbopol® 941 | | 1,25 |
| eau + conservateur + triéthanolamine | qsp | 100. |

En application locale 2 fois par jour sur le bas du visage et le cou, ce produit de soin améliore et maintient la fermeté et la tonicité de la peau du visage et en atténue les rides et ridules.

Exemple 5

| Emulsion raffermissante pour le visage: | |
|---|---|
| propylèneglycol | 1 |
| glycérylstéarate PEG 100 | 2 |
| glycérylstéarate sodium lauryl sulfate | 4 |
| glycéryl stéarate | 3 |
| alcool cétylique | 1 |
| triglycéride caprilique/capric | 3 |
| octyl stéarate | 2 |
| huile de camélia | 2 |
| extrait méthanolique sec de Polygonatum selon l'exemple 1 | 0,4 |
| palmitate de vitamine A | 0,01 |
| glycocéramides de blé | 0,1 |
| acide lactique | 0,5 |
| Carbopol® 940 | 0,5 |
| eau + conservateur + parfums          qsq | 100. |

Cette émulsion s'applique matin et soir et redonne l'élasticité au visage qui présente alors une bonne tonicité et un aspect raffermi.

Exemple 6

| Emulsion fluide pour le corps : | |
|---|---|
| huile végétale | 2 |
| triglycéride caprylic/capric | 5 |
| phosphate de vitamine E | 0,3 |
| carbomer | 0,5 |
| gomme xanthane | 0,20 |
| acide hyaluronique en solution à 1% | 1 |
| extrait de Polygonatum suivant exemple 1 | 0,2 |
| extrait d'Eriobotrya japonica | 0,1 |
| extrait de Centella asiatica | 0,1 |
| extrait de thé vert | 0,05 |
| eau + conservateur + agent rafraîchissant + parfum      qsp | 100 |

En application le soir, par cures de 20 jours, avec massage pour permettre aux tissus de conserver leur élasticité.

Exemple 7

| Lotion tonique pour le visage | |
|---|---|
| Lotion A | |
| extrait hydroéthanolique sec de Polygonatum selon l'exemple 2 | .. 0,3 |
| extrait d'Eriobotrya japonica | 1 |
| ginsénoside $R_o$ | 0,4 |
| solution aqueuse à 1% d'acide hyaluronique | 1 |
| glycérol | 2 |
| eau + conservateur          qsp | 100. |

Cette lotion tonique peut être utilisée par les femmes après le démaquillage ou par les hommes après le rasage.

**Revendications**

**1.** Utilisation cosmétique d'un extrait de polygonatum comme agent stimulant la synthèse d'élastine par les fibroblastes du derme, pour améliorer l'élasticité et la tonicité cutanée et favoriser le raffermissement de la peau, ledit agent étant incorporé dans une composition cosmétique incorporant un véhicule cosmétiquement acceptable.

**2.** Utilisation selon la revendication 1, **caractérisée en ce que** ledit extrait de Polygonatum est choisi parmi les extraits de rhizomes de Polygonatum kingianum, de P. sibiricum, de P. cyrtonema , de P. zanlanscianense et les autres extraits de rhizomes de Polygonatum connus sous le nom chinois de Huang Jing.

**3.** Utilisation selon l'une des revendications 1 ou 2, **caractérisée en ce que** ledit extrait est obtenu par un procédé mettant en oeuvre au moins une étape d'extraction de la matière végétale par un solvant polaire ou un mélange de solvants polaires.

**4.** Utilisation selon la revendication 3, **caractérisée en ce que** ledit solvant polaire ou mélange de solvants polaires est choisi dans le groupe constitué par l'eau, les alcools comprenant de préférence de 1 à 4 atomes de carbone, de préférence le méthanol, l'éthanol, le propanol, l'isopropanol, le propylèneglycol ou le butylèneglycol et les solvants mixtes à base de mélanges quelconques de ces solvants.

**5.** Utilisation selon la revendication 3 ou 4, **caractérisée en ce que** ledit solvant ou mélange de solvants polaires est choisi dans le groupe constitué par le méthanol, l'éthanol, le 1,3-butylèneglycol et les mélanges de ces solvants entre eux ou avec l'eau.

**6.** Utilisation selon l'une des revendications 1 à 5, **caractérisée en ce que** l'extrait de Polygonatum est utilisé par voie topique à une concentration comprise entre 0,001 % et 10 % en poids par rapport au poids total d'une composition le contenant dans un excipient, véhicule ou support approprié, en particulier cosmétiquement acceptable.

**7.** Utilisation selon la revendication 6, **caractérisée en ce que** l'extrait de Polygonatum précité est utilisé à une concentration comprise entre 0,01 et 2 % en poids par rapport au poids total de ladite composition.

**8.** Utilisation selon l'une des revendications 1 à 7, **caractérisée en ce que** l'extrait de Polygonatum précité est utilisé en combinaison avec une quantité efficace d'un principe actif stimulant la synthèse des composants de la matrice extracellulaire du derme, en particulier le collagène, en particulier le collagène I et le collagène III, et les glycosaminoglycanes, en particulier l'acide hyaluronique.

**9.** Utilisation selon la revendication 8, **caractérisée en ce que** l'extrait de Polygonatum précité est utilisé en combinaison avec un principe actif stimulant la synthèse du collagène, en particulier du collagène I et du collagène III, choisi parmi le groupe constitué par le madécassoside, un extrait de Centella asiatica, et un extrait de ginseng, en particulier contenant le ginsénoside $R_o$, à une concentration en poids de 0,01 % à 5 %.

**10.** Utilisation selon la revendication 8, **caractérisée en ce que** l'extrait de Polygonatum précité est utilisé en combinaison avec un principe actif stimulant la synthèse des glycosaminoglycanes, choisi dans le groupe constitué par les extraits de Filicium décipiens, d'Eriobotrya japonica, et le PDGF (platelet derived growth factor).

**11.** Utilisation selon l'une quelconque des revendications 1 à 10, **caractérisée en ce que** l'extrait de Polygonatum précité est utilisé en combinaison avec des vitamines, en particulier la vitamine A, son ester palmitique, propionique, ou acétique, ou la vitamine E et ses dérivés, en particulier son phosphate, à une concentration en poids de 0,0001 à 10 %.

**Claims**

**1.** Cosmetic use of an extract of Polygonatum as an agent stimulating the synthesis of elastin by the fibroblasts of the dermis, to improve the skin elasticity and tonicity and to promote skin firming.

**2.** Use according to claim 1, **characterised in that** said extract of Polygonatum is selected from the extracts of the rhizomes of Polygonatum kingianum, of P. sibiricum, of P. cyrtonema , of P. zanlanscianense and other extracts of the rhizomes of Polygonatum known under the Chinese name of Huang Jing.

**3.** Use according to one of claims 1 or 2, **characterised in that** said extract is obtained by a method using at least one step of extraction of the vegetable matter with a polar solvent or a mixture of polar solvents.

**4.** Use according to claim 3, **characterised in that** said polar solvent or mixture of polar solvents is selected from the group consisting of water, alcohols comprising preferably 1 to 4 carbon atoms, preferably methanol, ethanol, propanol, isopropanol, propyleneglycol or butyleneglycol and mixed solvents based on any mixture of these solvents.

**5.** Use according to claim 3 or 4, **characterised in that** said solvent or mixture of polar solvents is selected from the group consisting of methanol, ethanol, 1,3-butyleneglycol and mixtures thereof or mixtures of these solvents with water.

**6.** Use according to one of claims 1 to 5, **characterised in that** the extract of Polygonatum is used via the topical route at a concentration between 0.001 % and 10 % by weight with respect to the total weight of a composition containing same in a suitable excipient, vehicle or support, particularly cosmetically acceptable.

**7.** Use according to claim 6, **characterised in that** the extract of Polygonatum mentioned above is used at a concentration between 0.01 and 2 % by weight with respect to the total weight of said composition.

**8.** Use according to one of claims 1 to 7, **characterised in that** the extract of Polygonatum mentioned above is used in combination with an effective amount of an active principle stimulating the synthesis of the components of the extracellular matrix of the dermis, particularly collagen, particularly collagen I and collagen III, and glycosaminoglycans, particularly hyaluronic acid.

**9.** Use according to claim 8, **characterised in that** the extract of Polygonatum mentioned above is used in combination with an active principle stimulating the synthesis of collagen, particularly of collagen I and of collagen III, selected from the group consisting of madecassoside, an extract of Centella asiatica, and an extract of ginseng, particularly containing ginsenoside $R_o$, at a concentration by weight of 0.01 % to 5 %.

**10.** Use according to claim 8, **characterised in that** the extract of Polygonatum mentioned above is used in combination with an active principle stimulating the synthesis of glycosaminoglycans, selected from the group consisting of extracts of Filicium decipiens, of Eriobotrya japonica, and PDGF (platelet derived growth factor).

**11.** Use according to any one of claims 1 to 10, **characterised in that** the extract of Polygonatum mentioned above is used in combination with vitamins, particularly vitamin A, its palmitic, propionic or acetic ester, or vitamin E and its derivatives, particularly its phosphate, at a concentration by weight of 0.0001 to 10 %.

**Patentansprüche**

**1.** Kosmetische Verwendung eines Polygonatumextraktes als Mittel zur Stimulation der Elastinsynthese durch die Fibroblasten der Lederhaut, um die Hautelastizität und -spannkraft zu verbessern und um die Festigung der Haut zu begünstigen, wobei das Mittel einer kosmetischen Zusammensetzung beigemengt ist, welche ein kosmetisch akzeptables Vehikel enthält.

2. Verwendung nach Anspruch 1, **dadurch gekennzeichnet, dass** der Polygonatumextrakt ausgewählt ist unter den Polygonatum kingianum-, P. sibiricum-, P. cyrtonema-, P. zanlanscianense-Rhizom-Extrakten und den anderen Polygonatum-Rhizom-Extrakten, welche unter dem chinesischen Namen Huang Jing bekannt sind.

3. Verwendung nach einem der Ansprüche 1 oder 2, **dadurch gekennzeichnet, dass** der Extrakt durch ein Verfahren erhalten wird, das wenigstens einen Schritt zum Extrahieren des Pflanzenstoffes durch ein polares Lösungsmittel oder eine Mischung aus polaren Lösungsmitteln vollzieht.

4. Verwendung nach Anspruch 3, **dadurch gekennzeichnet, dass** das polare Lösungsmittel oder die Mischung aus polaren Lösungsmitteln ausgewählt ist aus der Gruppe bestehend aus Wasser, Alkoholen mit vorzugsweise 1 bis 4 Kohlenstoffatomen, vorzugsweise Methanol, Ethanol, Propanol, Isopropanol, Propylenglykol oder Butylenglykol und gemischten Lösungsmitteln auf der Basis von beliebigen Mischungen dieser Lösungsmittel.

5. Verwendung nach Anspruch 3 oder 4, **dadurch gekennzeichnet, dass** das Lösungsmittel oder die Mischung aus polaren Lösungsmitteln ausgewählt ist aus der Gruppe bestehend aus Methanol, Ethanol, 1,3-Butylenglykol und Mischungen dieser Lösungsmittel miteinander oder mit Wasser.

6. Verwendung nach einem der Ansprüche 1 à 5, **dadurch gekennzeichnet, dass** der Polygonatumextrakt örtlich in einer Konzentration zwischen 0,001 Gew.-% und 10 Gew.-% gegenüber dem Gesamtgewicht einer Zusammensetzung verwendet wird, die ihn in einem geeigneten, insbesondere kosmetisch akzeptablen Bindemittel, Vehikel oder Träger enthält.

7. Verwendung nach Anspruch 6, **dadurch gekennzeichnet, dass** der vorgenannte Polygonatumextrakt in einer Konzentration zwischen 0,01 und 2 Gew.-% gegenüber dem Gesamtgewicht der genannten Zusammensetzung verwendet wird.

8. Verwendung nach einem der Ansprüche 1 bis 7, **dadurch gekennzeichnet, dass** der vorgenannte Polygonatumextrakt in Verbindung mit einer wirksamen Menge eines aktiven Bestandteils verwendet wird, welcher die Synthese der Komponenten der extrazellulären Matrix der Lederhaut, insbesondere das Kollagen, insbesondere Kollagen I und Kollagen III, und die Glykosaminoglykane, insbesondere die Hyaluronsäure, stimuliert.

9. Verwendung nach Anspruch 8, **dadurch gekennzeichnet, dass** der vorgenannte Polygonatumextrakt in Verbindung mit einem aktiven, die Synthese des Kollagen, insbesondere des Kollagen I und des Kollagen III stimulierenden Bestandteil verwendet wird, welcher ausgewählt ist aus der Gruppe bestehend aus Madecassosid, einem Centella asiatica-Extrakt und einem Ginseng-Extrakt, insbesondere enthaltend Ginsenosid $R_o$, in einer Gewichtskonzentration von 0,01 % bis 5%.

10. Verwendung nach Anspruch 8, **dadurch gekennzeichnet, dass** der vorgenannte Polygonatumextrakt in Verbindung mit einem aktiven, die Synthese der Glykosaminoglykane stimulierenden Bestandteil verwendet wird, welcher ausgewählt ist aus der Gruppe bestehend aus Filicium decipiens-, Eriobotrya japonica-Extrakten und PDGF (platelet derived growth factor).

11. Verwendung nach irgendeinem der Ansprüche 1 bis 10, **dadurch gekennzeichnet, dass** der vorgenannte Polygonatumextrakt in Verbindung mit Vitaminen, insbesondere Vitamin A, dessen Palmitin-, Propion- oder Essigester, oder Vitamin E und dessen Derivaten, insbesondere dessen Phosphat, in einer Gewichtskonzentration von 0,0001 bis 10%, verwendet wird.